(19) Europäisches Patentamt
European Patent Office
Office européen des brevets

(11) Publication number: 0 222 257
A2

# (12) EUROPEAN PATENT APPLICATION

(21) Application number: 86115012.6

(22) Date of filing: 29.10.86

(51) Int. Cl.4: A61K 31/66 , A61K 9/00 , C07F 9/22

(30) Priority: 11.11.85 IT 2277385

(43) Date of publication of application:
20.05.87 Bulletin 87/21

(84) Designated Contracting States:
AT BE CH DE ES FR GB GR IT LI LU NL SE

(71) Applicant: SCHIAPPARELLI FARMACEUTICI S.p.A.
P.zza Duca d'Aosta, 12
I-20124 Milano(IT)

(72) Inventor: Cannata, Vincenzo
Via Annibale Ciò, 12
I-40044 Borgo Nuovo, Pontecchio Marconi(IT)
Inventor: Mardente, Salvatore
Via Vezzolano, 21
I-10153 Torino(IT)

(74) Representative: Bellenghi, Mario Dr. et al
Ing. A. Giambrocono & C. s.r.l. Via Rosolino Pilo, 19/B
I-20129 Milano(IT)

## (54) A phosphocreatine containing pharmaceutical composition and method for preparing the same.

(57) A glycine-free, phosphocreatine containing pharmaceutical composition, and a method for preparing crystalline phosphocreatine suitable for administration in high doses.

EP 0 222 257 A2

# A PHOSPHOCREATINE CONTAINING PHARMACEUTICAL COMPOSITION AND METHOD FOR PREPARING THE SAME.

## Background of the invention

Phosphocreatine as such or in the form of its alkali metal salts has found pharmacological and therapeutical use in pathologic conditions of striated musculature, such as muscular atrophy and dystrophy, and also of heart musculature, such as myocardiosclerosis, degenerative myocardiopathies and, more generally, anoxies, i.e. those conditions in which the myocardial contractility must be restored as quickly as possible.

The dosages at which phosphocreatine, as such or in the form of its alkali metal salts, displays said biological properties in animals, for instance in dogs, generally range between about 0.5 mg/kg and about 6 mg/kg.

In therapy, they are administered to patients suffering from the above mentioned pathologic conditions, by intramuscular or intravenous route in a daily dosage ranging between about 200 and about 400 mg. The pharmaceutical preparations contain the active ingredient in an amount of about 200 mg, in combination with the usually employed pharmaceutical carriers.

## Disclosure of the invention

This invention is concerned with new pharmaceutical compositions containing phosphocreatine, chemically N-(alpha-imino-alpha--phosphonamido)-methyl-N-methylglycine

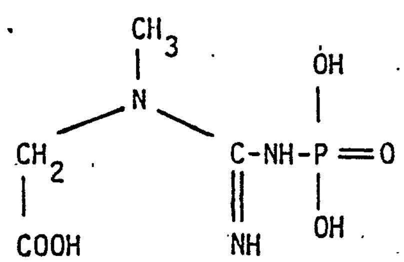

Particularly, this invention concerns glycine-free, phosphocreatine containing pharmaceutical compositions wherein phosphocreatine is crystallized in such a form that its administration is easy. The invention also concerns a process for the preparation of said crystalline form of phosphocreatine.

Phosphocreatine is a well known natural substance. It is essentially distributed within several tissues of vertebrates, particular within the skeleton muscles. It was first isolated from frog muscles - (Eggleton, Biochem. J. 21, 190, 1927) and cat muscles (Fiske et al., J. Biol. Chem. 81, 629, 1929). Its chemical synthesis by phosphorylation of creatine was first described by Zeile et al., J. Physiol. Chem. 256 193, 1938; Ennor et al., Biochem. J. 43 190, 1948: Ennor et al., Phisiol. Rev. 38 631, 1938. A more recent chemical synthesis is described in Italian Patent No. 1,044,765.

Phosphocreatine is formed in muscular tissue by phosphorylation of creatine. It has a fundamental role in maintaining a valid muscle contraction, a physico-chemical phenomenon conditioned by energy utilization. It is known that the muscular contraction is caused by the release of a phosphate radical by adenosine triphosphate (ATP) which is converted to adenosine diphosphate (ADP).

This latter needs more phosphoric acid in order to renegerate ATP, which therefore represents the energy supply necessary for maintaining the ability of a valid muscle contraction.

Under rest conditions, the regeneration of ATP consumed in basal activity occurs at the expenses of those physiological processes which are in condition to supply phosphoric radicals with high energy contents. These radicals derive from the metabolic breakdown of glycogen contained in muscles.

Under activity conditions the necessary energetic requirement is substantially increased, and becomes insufficient. In these circumstances the ready supply of phosphoric radicals with high energetic level is provided for by the phosphocreatine present in muscle. Phosphocreatine is synthesized through the same metabolic mechanism which leads to the synthesis of ATP (cleavage of glucids with formation of two phosphocreatine moles for each mole of formed lactic acid).

It is apparent that the hexogenic phosphocreatine supply allows to save all or most of the metabolic work of glucid breakdown which, as above pointed out, is necessary to ensure the supply of phosphoric radicals with high potential level both to ATP and to phosphocreatine.

Phosphocreatine, as such or in the form of its alkali metal salts, has found pharmacological and therapeutical use in pathologic conditions involving striated musculature (for instance muscular atrophies and dystrophies) and also heart muscle:

myocardiosclerosis, degenerative myocardiopathies and, more generally, anoxies, i.e. those conditions in which it is necessary to restore myocardial contractility as soon as possible.

Dosages at which phosphocreatine, as such or in the form of the corresponding alkali metal salts, exerts these biological properties in animals, e.g. in dogs, generally range between about 0.5 and about 6 mg/kg. In therapy, they are administered to patients suffering from the above mentioned pathological conditions, intramuscularly or intravenously in daily dosages ranging between about 200 and about 400 mg/kg.

The usual pharmaceutical compositions contain the active ingredient in an amount of about 200 mg, combined with the usually employed pharmaceutical carriers.

It has been now surprisingly found that phosphocreatine, as such or in the form of its salts with alkali metals, when administered at doses substantially higher than those at which it displays its activity on striated muscles, exerts a stabilizing activity on the sarcolemmatic membrane of myocardial cells, thus protecting said membrane if used in cardioplegic solutions in acute myocardial ischemia.

During research carried out in recent years it was apparent that at dosages higher than those usually employed, phosphocreatine causes other mechanisms beyond the postulated intervention in Lohmann's reaction.

Electronic microscopy studies carried out using colloidal lanthanum as tracer, both in animals and humans, showed a remarkable stabilization of ischemic cardiomyocytes.

Colloidal lanthanum, ultrastructural marker of early sarcolemma damage, does not penetrate normal heart cells and accumulates within the extracellular space. In ischemic conditions, on the contrary, sarcolemma of cardiomyocytes becomes permeable to colloidal lanthanum which may be put into evidence by the ultrastructural examination in relation with mitochondrial membranes.

In the presence of 10 mM concentration of phosphocreatine, the sarcolemma of cardiomyocytes remains impermeable to colloidal lanthanum also under conditions of severe experimental ischemia. This shows a protective effective of phosphocreatine on the integrity of the cell membrane of ischemic cardiomyocytes.

This effect was concurrently observed in tests carried out under different experimental conditions, such as experimental infarction following coronary ligature in rabbits and dogs, total ischemia of slices of papillar muscle of mini-pig in vitro heart, surgery in man.

Similar conclusions are reached by examining other direct and indirect indexes of integrity of cell membrane, such as ultrastructural morphologic examination and enzymatic assays (CPL release).

The importance of this finding becomes apparent if one considers that sarcolemma is the most sensitive cardiac cell structure and the first one to undergo changes under ischemic conditions.

Therefore, to protect sarcolemma from the ischemic damage means to prevent a number of events leading to irreversible cell damage, with untoward effect on myocardium metabolism, its morphological integrity, contractile functionality, electrophysiology.

The foregoing considerations allow an interpretation of phosphocreatine effects in the various experiments of myocardial protection in vitro and in vivo in animals and humans, consisting in a better morphologic, biochemical, functional (contractile ability), and electrical (lower occurence of arrhythmias) recovery.

An interpretation on biochemical basis of this membrane stabilizing effect was suggested in studies of membrane phospholipid metabolism on heart myocardiocytes of dogs with coronary ligation. The conclusions derived from these studies were that the membrane stabilizing effect of phosphocreatine may, at least partially, be attributed to an inhibitory action on the phospholipide breakdown of the cell membrane of myocardiocytes which are normally degraded to lysophosphatides.

As briefly mentioned above, recent clinical researches in the field of human cardioplegy and myocardial protection during acute ischemia showed markedly positive effects of phosphocreatine at doses of 2-3 g/day intramuscularly, 2-8 g/day intravenously and 8-10 g in cardioplegic solutions.

These doses are much higher than those used sofar. This creates the problem of preparing new formulations with higher phosphocreatine doses - (0.5-1 g and more) useful for intramuscular and/or intravenous administration.

The methods for the preparation of formulations sofar employed in clinics involves the presence of high amounts of glycine,which is necessary as support in the lyophilization step.

In lyophilized phosphocreatine ampoules with 200 mg doses of active ingredient, glycine is present in an amount of 250 mg. Proportionally higher glycine amounts would be preparing present in ampoules with higher phosphocreatine doses.

The presence of glycine causes a marked increase of osmoticity of injectable solutions with problems of topical tolerance.

A further advantage of using phosphocreatine prepared by the process of the present invention consists in the fact that phosphocreatine is in the form of a crystalline powder with about 30 percent of crystallization water, corresponding to about 6 molecules of crystallization water per phosphocreatine molecule. This imparts to the substance a low degree of hygroscopicity and a better stability and longer storage life.

The clinical requirement of parenteral phosphocreatine formulations with doses of 0.5 grammes and more, involves therefore a parallel increase of glycine amount. The end result is a solution of undesirably high volume and osmoticity, not topically tolerable , and unfit for intramuscular administration. Moreover, intravenous administration of very high glycine doses may cause pharmacological interferences and pharmacodynamic effects of which this amino acid is responsible.

In order to administer phosphocreatine doses having heart protecting activity in conformity with the results of the recent clinical research, a new method of preparation was therefore required, with the exclusion of ingredients, particularly glycine, and decreasing both the osmoticity and the volume of the end solution. This was the only way for preparing formulations containing 0.5-1 grammes and more of active substance, and having osmoticity and volume compatible with the intramuscular administration and preventing possible pharmacological interference by glycine when administering still higher intravenous doses or solutions for cardiac infusion during surgical heart interventions.

The method for the preparation of phosphocreatine in a form useful for parenteral administration in high doses and in glycine free formulations consists in dissolving 1 parts in weight of sodium phosphocreatine in 1-2 parts in weight of water, cooling the thus obtained solution to a temperature between about 0° and about 10°C, and adding gradually in a period of 1 to 3 hours, preferably about 2 hours, a volume of ethanol corresponding to 2-3 times the volume of water in which sodium phosphocreatine is dissolved. The sodium salt precipitates in a crystalline form. It is scarcely hygroscopic, is quickly and completely soluble in water and is therefore suitable for the extemporaneous preparation of injectable solutions for intravenous administration or heart infusion during heart surgery.

The following Examples are illustrative of the present invention, but are not intended to establish its limits.

EXAMPLE 1

Preparation of crystalline sodium phosphocreatine.

In an appropriate container provided with thermometer and dropping funnel, 500 g of sodium phosphocreatine and 650 ml of water are stirred until complete solution is obtained. The solution is filtered through millipore filter of 0.22 microns.

The filtered solution is charged again into the reaction container, cooled to 0-2°C, then 1500 ml of 95 percent ethanol, previously filtered through millipore, is slowly added with stirring in about 2 hours.

A precipitate is gradually formed and when all ethanol has been added, crystallization is substantially complete. Stirring is continued for an additional hour, then the solid is collected on a filter plate in vacuo.

The collected product is dried in vacuo at 40°C. Yield 470 g of crystalline sodium phosphocreatine.

All the foregoing steps are carried out under sterile conditions, thus obtaining a sterile product suitable for parenteral administration.

EXAMPLE 2

A formulation for parenteral use is prepared from 1 bottle containing 500 mg of crystalline sodium phosphocreatine; 1 ampoule containing 4 ml of sterile distilled water.

The contents of the ampoule is withdrawn at the moment of use and introduced into the bottle containing sodium phosphocreatine to have a 12.5 percent solution of active ingredient.

EXAMPLE 3

By the same procedure of Example 2 a bottle with 1 g of crystalline sodium phosphocreatine and an ampoule with 4 ml of sterile distilled water are prepared. The end solution ready for use has a 25 percent concentration of active substance.

EXAMPLE 4

A 1 percent solution of sodium phosphocreatine is prepared by admixing the contents of a bottle containing 1 g of crystalline sodium phosphocreatine and of an ampoule containing 100 ml of sterile distilled water. The solution is suitable for administration by phleboclysis.

EXAMPLE 5

By the same procedure of Example 4, a 5 percent solution for phleboclysis is prepared.

EXAMPLE 6

By the same procedure of Example 4, a 10 percent solution for phleboclysis is prepared.

## Claims

1.-A composition for parenteral use prepared by employing sodium phosphocreatine in crystalline form.

2.-A composition for parenteral use containing 1-25 weight percent of sodium phosphocreatine dissolved in an injectable solvent, characterized by the fact that crystalline sodium phosphocreatine is used for the preparation of said composition.

3.-A composition for parenteral use as claimed in claim 2, characterized by the fact that the solvent is sterile distilled water.

4.-A pharmaceutical formulation for parenteral use consisting of

a) a bottle containing 0.5-1 g of crystalline sodium phosphocreatine and

b) an ampouble containing 4 ml of sterile distilled water the ampoule contents being added to the bottle contents at the moment of us to obtain a sterile sodium phosphocreatine solution suitable for parenteral administration.

5.-A pharmaceutical formulation of parenteral use consisting of

a) a bottle containing 0.1-10 g of crystalline sodium phosphocreatine and

b) an ampoule containing 100 ml of sterile distilled water the ampoule contents being added to the bottle contents at the moment of use to obtain a sterile sodium phosphocreatine solution suitable for phleboclysis.

6.-A pharmaceutical formulation as claimed in any of claims 1 to 5, characterized by the fact that it is prepared using sterile procedures and materials to obtain a solution suitable for parenteral administration.

7.-A process for preparing crystalline sodium phosphocreatine suitable for the preparation of high dose, glycine free parenteral solutions, which comprises dissolving 1 part in weight of sodium phosphocreatine in 1-2 parts in weight of water, cooling the obtained solution to a temperature between 0°C and 10°C, adding to said solution, in a period of 1-3 hours, 2-3 parts in volume of ethanol for each part in volume of said solution and collecting the precipitated crystalline sodium phosphocreatine.